# FASCICULE DE BREVET EUROPEEN

(11) **EP 1 092 425 B1**
(45) Date de publication et mention de la délivrance du brevet: **05.06.2002**
(21) Numéro de dépôt: 00402354.5
(22) Date de dépôt: 24.08.2000
(51) Int. Cl.: A61K 7/48, A61K 7/06, A61K 7/02

(54) **Composition sous forme d'émulsion eau-dans-huile, contenant des fibres, et son utilisation dans le domaine cosmétique**
Faserhaltige Wasser-in-Öl Zusammensetzung und ihre Verwendung im kosmetischen Bereich
Water-in-oil composition containing fibers, and its use in the cosmetic area

(30) Priorité: 15.10.1999 FR 9912911
(43) Date de publication de la demande: 18.04.2001
(73) Titulaire: L'OREAL, 75008 Paris (FR)
(72) Inventeur: Afriat, Isabelle, 75003 Paris (FR)
(74) Mandataire: Rasson, Catherine

(56) Documents cités:
- EP-A- 0 838 210
- FR-A- 2 776 183
- US-A- 4 659 562
- PATENT ABSTRACTS OF JAPAN vol. 1999, no. 01, 29 janvier 1999 (1999-01-29) & JP 10 287523 A (POLA CHEM IND INC), 27 octobre 1998 (1998-10-27)
- PATENT ABSTRACTS OF JAPAN vol. 012, no. 110 (C-486), 8 avril 1988 (1988-04-08) & JP 62 238211 A (KOBAYASHI KOOC:KK), 19 octobre 1987 (1987-10-19)

## Description

L'invention se rapporte à une composition se présentant sous forme d'une émulsion eau-dans-huile (E/H) contenant des fibres, un tensioactif siliconé et une cire, et à l'utilisation de la dite composition, en particulier pour le soin, le traitement et/ou le maquillage de la peau du corps ou du visage, des cheveux, des cils et/ou des lèvres.

Il est connu par le document JP07-196440 des compositions cosmétiques contenant des fibres de polyamide courtes, celles-ci donnant aux dites compositions un toucher velouté et une bonne tenue cosmétique. Toutefois, l'incorporation de ces fibres de polyamide dans les émulsions eau dans huile (E/H) pose des problèmes de stabilité, c'est-à-dire que les émulsions déphasent à température ambiante ou à températures plus élevées, et ce notamment quand la quantité de fibres est importante.

Il subsiste donc le besoin d'émulsions E/H contenant des fibres, qui soient stables tout en présentant de bonnes propriétés cosmétiques et donc qui n'aient pas les inconvénients de celles de l'art antérieur.

La demanderesse a découvert de façon inattendue que l'utilisation d'un tensioactif siliconé associé à une cire permettait de réaliser des émulsions eau-dans-huile contenant des fibres, stables et cosmétiquement agréables, à savoir douces et non grasses. En outre, on peut incorporer dans les compositions selon l'invention, une quantité de fibres plus importante que décrit dans le document JP07-196440.

L'invention a pour objet une composition sous forme d'émulsion comprenant, dans un milieu physiologiquement acceptable, une phase aqueuse dispersée dans une phase huileuse, caractérisée en ce qu'elle contient des fibres, au moins un tensioactif siliconé et au moins une cire.

On entend par « milieu physiologiquement acceptable » un milieu compatible avec la peau, les lèvres, les ongles, le cuir chevelu et/ou les cheveux.

La composition obtenue selon l'invention présente une bonne stabilité dans le temps, même à une température supérieure à la température ambiante (par exemple 45°C). Cette composition a l'aspect d'une crème (produit souple par opposition à un produit solide) et elle a une texture veloutée, agréable à l'application.

Les fibres utilisables dans la composition de l'invention peuvent être des fibres d'origine synthétique ou naturelle, minérale ou organique. Elles peuvent être courtes ou longues, unitaires ou organisées par exemple tressées. Leur forme peut être quelconque et notamment de section circulaire ou polygonale (carrée, hexagonale ou octogonale) selon l'application spécifique envisagée. En particulier, leurs extrémités sont épointées et/ou polies pour éviter de se blesser.

En particulier, les fibres ont une longueur allant de 1 nm à 20 mm, de préférence de 10 nm à 5 mm et mieux de 0,1 mm à 1,5 mm. Leur section peut être comprise dans un cercle de diamètre allant de 2 nm à 100 µm, de préférence allant de 20 nm à 20 µm et mieux de 5 µm à 50 µm. Le poids des fibres est souvent donné en denier ou décitex.

Les fibres peuvent être celles utilisées dans la fabrication des textiles et notamment des fibres de soie, de coton, de laine, de lin, de cellulose extraites notamment du bois, des légumes ou des algues, de polyamide (Nylon®), de rayonne, de viscose, d'acétate notamment d'acétate de rayonne, d'acétate de cellulose ou d'acétate de soie, de poly-p-phénylène téréphtamide notamment de Kevlar®, en acrylique notamment de polyméthacrylate de méthyle ou de poly-2-hydroxyéthylméthacrylate, de polyoléfine et notamment de polyéthylène ou de polypropylène, de verre, de silice, d'aramide, de carbone notamment sous forme graphite, de Téflon®, de collagène insoluble, de polyesters, de polychlorure de vinyle ou de vinylidène, d'alcool polyvinylique, de polyacrylonitrile, de chitosane, de polyuréthane, de polyéthylène phtalate, des fibres formées d'un mélange de polymères tels que ceux mentionnés ci-avant, comme des fibres de polyamide/polyester, et les mélanges de ces fibres.

On peut aussi utiliser les fibres chirurgicales comme les fibres synthétiques résorbables préparées à partir d'acide glycolique et de caprolactone (« Monocryl » de la société Johnson & Johnson) ; les fibres synthétiques résorbables du type copolymère d'acide lactique et d'acide glycolique (« Vicryl » de la société Johnson & Johnson) ; les fibres de polyester téréphtalique (« Ethibond » de la société Johnson & Johnson) et les fils d'acier inoxydable (« Acier » de la société Johnson & Johnson).

Par ailleurs, les fibres peuvent être traitées ou non en surface, enrobées ou non. Comme fibres enrobées utilisables dans l'invention, on peut citer des fibres de polyamide enrobées de sulfure de cuivre pour un effet antistatique (par exemple les fibres R-STAT de la société Rhodia) ou un autre polymère permettant une organisation particulière des fibres (traitement de surface spécifique) ou traitement de surface induisant des effets de couleurs/hologrammes (fibre « Lurex » de la société Sildorex, par exemple).

Les fibres utilisables dans la composition selon l'invention sont de préférence des fibres de polyamide ou de poly-p-phénylène téréphtamide. Leur longueur peut aller de 0,1 à 5 mm, de préférence de 0,25 à 1,6 mm, et leur diamètre moyen peut aller de 5 à 50 µm. En particulier, on peut utiliser les fibres de polyamide commercialisées par les Etablissements P. Bonte sous le nom Polyamide 0.9 Dtex 0.3 mm, ayant un diamètre moyen de 6 µm, un poids d'environ 0.9 dtex et une longueur allant de 0,3 mm à 1,5 mm. On peut aussi utiliser les fibres de poly-p-phénylène téréphtamide de diamètre moyen de 12 µm et de longueur d'environ 1,5 mm comme celles vendues sous le nom de Kevlar Floc par la société Du Pont Fibres.

Les fibres peuvent être présentes dans la composition selon l'invention en une quantité allant de 0,1 à 20 % en poids et de préférence de 0,5 à 12 % en poids par rapport au poids total de la composition.

Comme tensioactifs siliconés pouvant entrer dans la composition selon l'invention, on peut citer les dimethicone copolyols et les alkyldiméthicone copolyols. Comme dimethicone copolyol, on peut citer par exemple le mélange de dimethicone copolyol, de cyclomethicone et d'eau (10/88/2), commercialisé par la société Dow Corning sous la dénomination DC3225C ou DC2-5225C, et le mélange de dimethicone copolyol et de cyclopentasiloxane (85/15) commercialisé sous la dénomination Abil EM-97 par la société Goldschmidt. Comme alkyldiméthicone copolyol, on peut citer notamment ceux ayant un radical alkyle comportant de 10 à 22 atomes de carbone, tel que le cétyl diméthicone copolyol comme le produit commercialisé sous la dénomination Abil EM-90 par la société Goldschmidt ; le lauryl diméthicone copolyol et par exemple le mélange d'environ 91 % de lauryl diméthicone copolyol et d'environ 9 % d'alcool isostéarylique, commercialisé sous la dénomination Q2-5200 par la société Dow Corning, et les mélanges de ces tensioactifs siliconés. Le tensioactif siliconé est de préférence un alkyldiméthicone copoyol et notamment le cétyldiméthicone copolyol.

La quantité de tensioactif(s) siliconé(s) dans la composition de l'invention va de préférence de 0,1 à 5 % en poids de matière active, et mieux de 0,5 à 2 % en poids de matière active par rapport au poids total de la composition.

La composition selon l'invention comprend au moins une cire habituellement présente dans la phase huileuse de l'émulsion. Une cire a généralement une température de fusion commençante supérieure ou égale à 45°C. Par "température de fusion commençante", on entend dans la présente description la température à laquelle une cire commence à fondre. On peut déterminer cette température par ATD (analyse thermique différentielle) qui permet l'obtention du thermogramme (ou courbe de fusion) de la cire considérée. La température de fusion commençante correspond à la température à laquelle on peut observer un changement de pente notable dans le thermogramme. Le point de fusion, quant à lui, représente le point minimum dudit thermogramme.

A titre d'exemples de cires utilisables dans la composition de l'invention, on peut citer les cires minérales telles que les cires microcristallines, la paraffine, le pétrolatum, la vaseline, l'ozokérite, la cire de montan ; les cires animales telles que la cire d'abeilles, la lanoline et ses dérivés ; les cires végétales telles que les cires Candellila, d'Ourrury, de Carnauba, du Japon, le beurre de cacao, les cires de fibres de lièges ou de canne à sucre ; les huiles hydrogénées concrètes à 25°C telles que l'huile de jojoba hydrogénée ; les esters gras et les glycérides concrets à 25°C ; les cires synthétiques telles que les cires de polyéthylène et les cires obtenues par synthèse de Fischer-Tropsch ; les cires de silicone, et leurs mélanges.

On utilise de préférence comme cire, la cire de polyéthylène, l'huile de jojoba hydrogénée et leurs mélanges.

La quantité de cire(s) dans la composition de l'invention va de préférence de 0,5 à 10 % et mieux de 1,5 à 7 % en poids par rapport au poids total de la composition.

La phase huileuse de la composition selon l'invention peut renfermer, outre la ou les cires, toute sorte d'huiles et de corps gras bien connus de l'homme du métier, comme exemple les huiles d'origine végétale (jojoba, avocat, sésame, tournesol, maïs, soja, carthame, pépins de raisin), les huiles minérales (vaseline, isoparaffines éventuellement hydrogénées), les huiles de synthèse (myristate d'isopropyle, octanoate de cétéaryle, polyisobutylène, palmitate d'éthyl-hexyle, alkyl-benzoates), les huiles de silicone volatiles ou non volatiles, et les huiles fluorées ou fluorosiliconées, ainsi que les mélanges de ces huiles.

De préférence, la phase huileuse de la composition de l'invention comprend au moins une huile de silicone qui peut être présente en une quantité allant par exemple de 5 à 50 % en poids et de préférence de 9 à 30 % en poids par rapport au poids total de la composition. Comme huile de silicone, on peut citer par exemple les huiles de silicone volatiles telles que les cyclodiméthylsiloxanes ou cyclométhicones, comme la pentacyclométhicone, la tétracyclométhicone ou l'hexacyclométhicone; les huiles de silicone non volatiles telles que les polydiméthylsiloxanes (PDMS). De préférence, la composition de l'invention contient au moins une huile de silicone volatile.

La phase huileuse peut contenir, en outre, d'autres constituants gras tels que les alcools gras comme l'alcool stéarylique, l'alcool cétylique et l'alcool cétéarylique ; les acides gras ; les gommes et notamment les gommes de silicone comme le mélange PDMS à groupements alpha oméga hydroxylés / PDMS 5 cst (12/88) vendu sous la dénomination DC 1503 par la société Dow Corning.

La phase huileuse est présente dans la composition selon l'invention en une quantité allant généralement de 10 à 50 % et de préférence de 12 à 40 % en poids par rapport au poids total de la composition, cette quantité comprenant la quantité de tensioactif siliconé.

La phase aqueuse de la composition de l'invention peut aller de 30 à 85 % en poids et de préférence de 40 à 75 % en poids par rapport au poids total de la composition, et peut contenir, outre l'eau, des solvants tels que les alcools primaires comportant de 1 à 4 atomes de carbone comme l'éthanol, ou les polyols comme le butylène glycol. Le ou les solvants peuvent être présents en une quantité allant de 0,1 à 30 % en poids par rapport au poids total de la composition.

La composition de l'invention peut contenir aussi des gélifiants lipophiles tels que les argiles comme par exemple les bentones; les organopolysiloxanes élastomères comme par exemple ceux commercialisés sous les noms KSG 6 de Shin-Etsu, Trefil E-505C ou Trefil E-506C de Dow-Corning, Gransil (SR-CYC, SR DMF10, SR-DC556) de Grant Industries, ou ceux commercialisés sous forme de gels déjà constitués : KSG 15, KSG 17, KSG 16, KSG 18 de Shin-Etsu, Gransil SR 5CYC gel, Gransil SR DMF 10 gel, Gransil SR DC556 gel de Grant Industries, 1229-02-167 et 1229-02-168 de General Electric. On peut aussi utiliser un mélange de ces produits commerciaux.

La composition selon l'invention a l'aspect d'une crème et peut constituer notamment une compositon cosmétique ou dermatologique. Elle trouve alors son application dans un grand nombre de traitements notamment cosmétiques de la peau, y compris du cuir chevelu, des cheveux, des ongles, et/ou des muqueuses. en particulier pour le soin, le nettoyage, le maquillage et/ou la protection solaire de la peau et/ou des muqueuses.

Aussi, la présente invention a pour objet l'utilisation cosmétique de la composition telle que définie ci-dessus, pour le traitement, la protection, le soin, le démaquillage et/ou le nettoyage de la peau, des lèvres et/ou des cheveux, et/ou pour le maquillage de la peau et/ou des lèvres.

La présente invention a encore pour objet un procédé de traitement cosmétique de la peau, y compris du cuir chevelu, des cheveux, et/ou des lèvres, caractérisé par le fait que l'on applique sur la peau, les cheveux et/ou les lèvres, une composition telle que définie ci-dessus.

De façon connue, la composition de l'invention peut contenir également des adjuvants habituels dans les domaines cosmétique et/ou dermatologique, tels que les actifs, les conservateurs, les antioxydants, les agents complexants, les ajusteurs de pH (acides ou basiques), les parfums, les charges (polyéthylène), les bactéricides, les absorbeurs d'odeur, les matières colorantes (colorants et pigments) et encore les vésicules lipidiques. Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse, dans la phase aqueuse et/ou dans les vésicules lipidiques.

Comme actifs, on peut citer notamment les hydratants et par exemple les hydrolysats de protéines et les polyols tels que la glycérine, les glycols comme les polyéthylène glycols, et les dérivés de sucre.

Le ou les actifs peuvent être par exemple présents en une concentration allant de 0,01 à 20 %, de préférence de 0,1 à 5 % et mieux de 0,5 à 3 % du poids total de la composition.

Les exemples ci-après de compositions selon l'invention sont donnés à titre d'illustration et sans caractère limitatif. Les quantités y sont données en % en poids, sauf mention contraire.

### Exemple 1 : crème protectrice

### A. Phase huileuse

- Cétyldiméthicone copolyol / isostéarate de polyglycéryl-4/Hexyl laurate (ABIL WE 09 commercialisé par GOLDSCHMIDT) 4 %
- Huile de jojoba hydrogénée 5,2 %
- Huile de silicone volatile (cyclohexadiméthylsiloxane) 2,2 %
- Cire de polyéthylène 0,8 %
- Octanoate de cétarylelmyristate d'isopropyle (huiles) 7 %
- Nylon 12 0,8 %

### B. Phase aqueuse

- Chlorure de sodium 0,5 %
- Glycérine 2 %
- Eau qsp 100 %

### C. Empâtage

- Fibres de polyamide (Polyamide 0,9 Dtex, 0,3 mm - Société Paul Bonte) 8 %
- Gomme de silicone 1,3 %
- Trifluorométhyl alkyldiméthicone (X-22-819, Shin Etsu, mouillant) 1,6 %
- Pigments 0,1 %

Mode opératoire : on chauffe la phase huileuse sans la silicone volatile jusqu'à ce que celle-ci soit homogène. Par ailleurs, on chauffe la phase aqueuse à la même température. On prépare l'émulsion en versant la phase aqueuse dans la phase huileuse. Puis ajouter la silicone volatile. Puis on ajoute la phase C et on refroidit.

On obtient une crème teintée et protectrice.

### Exemple 2 : Crème hydratante

### A. Phase huileuse

- Cétyldiméthicone copolyol / isostéarate de polyglycéryl-4/Hexyl laurate (ABIL WE 09 commercialisé par GOLDSCHMIDT) 5 %
- Huile de jojoba hydrogénée 5,5 %
- Huile de silicone volatile (cyclohexadiméthylsiloxane) 7,3 %
- Cire de polyéthylène 0,8 %
- Octanoate de cétaryle/myristate d'isopropyle (huiles) 7 %
- Nylon 12 0,8 %

### B. Phase aqueuse

- Chlorure de sodium 0,5 %
- Glycérine 2 %
- Eau qsp 100 %

### C. Empâtage

- Fibres de polyamide (Polyamide 0,9 Dtex, 0,3 mm - Société Paul Bonte) 8 %
- Huile fluorosiliconée (fluoro-propyl dimethylsiloxane) (X-22-819 vendu par la société Shin Etsu) 1,6 %
- Organopolysiloxane réticulé à 24 % de matière active dans PDMS non volatil (KSG 16) 2,1 %

Mode opératoire : on chauffe la phase huileuse sans la silicone volatile jusqu'à ce que celle-ci soit homogène. Par ailleurs, on chauffe la phase aqueuse à la même température. On prépare l'émulsion en versant la phase aqueuse dans la phase huileuse. Puis ajouter la silicone volatile. Puis on ajoute la phase C et on refroidit.

On obtient une crème blanche apte à hydrater la peau.

### Exemple 3 : Emulsion coulée

### A. Phase huileuse

- Cétyldiméthicone copolyol / isostéarate de polyglycéryl-4/Hexyl laurate (ABIL WE 09 commercialisé par GOLDSCHMIDT) 5 %
- Huile de jojoba hydrogénée 5,5 %
- Huile de silicone volatile (cyclohexadiméthylsiloxane) 7,3 %
- Cire de polyéthylène 0,8 %
- Octanoate de cétaryle/myristate d'isopropyle (huiles) 7 %
- Bentone 2,1 %

### B. Phase aqueuse

- Chlorure de sodium 0,5 %
- Glycérine 2 %
- Eau qsp 100 %

### C. Empâtage

- Fibres de polyamide (Polyamide 0,9 Dtex, 0,3 mm - Société Paul Bonte) 8 %
- Huile fluorosiliconée (fluoro-propyl dimethylsiloxane) (X-22-819 vendu par la société Shin Etsu) 1,6 %
- Organopolysiloxane réticulé à 24 % de matière active dans PDMS non volatil (KSG 16) 2,1 %

Mode opératoire : on chauffe la phase huileuse sans la silicone volatile jusqu'à ce que celle-ci soit homogène. Par ailleurs, on chauffe la phase aqueuse à la même température. On prépare l'émulsion en versant la phase aqueuse dans la phase huileuse. Puis ajouter la silicone volatile. Puis on ajoute la phase C toujours sous agitation, et on coule immédiatement après dans un moule.

On obtient une crème blanche apte à protéger la peau.

## Revendications

1. Composition sous forme d'émulsion eau-dans-huile (E/H) comprenant, dans un milieu physiologiquement acceptable, une phase aqueuse dispersée dans une phase huileuse, **caractérisée en ce qu'**elle contient des fibres, au moins un tensioactif siliconé et au moins une cire.

2. Composition selon l'une des revendications précédentes, **caractérisée en ce que** les fibres sont choisies parmi les fibres de soie, de coton, de laine, de lin, de cellulose extraites notamment du bois, des légumes ou des algues, de polyamide, de rayonne, de viscose, d'acétate notamment d'acétate de rayonne, d'acétate de cellulose ou d'acétate de soie, de poly-p-phénylène téréphtamide, en acrylique notamment de polyméthacrylate de méthyle ou de poly-2-hydroxyéthylméthacrylate, de polyoléfine et notamment de polyéthylène ou de polypropylène, de verre, de silice, d'aramide, de carbone notamment sous forme graphite, de Téflon®, de collagène insoluble, de polyesters, de polychlorure de vinyle ou de vinylidène, d'alcool polyvinylique, de polyacrylonitrile, de chitosane, de polyuréthane, de polyéthylène phtalate, des fibres de mélanges de polymères, des fibres chirurgicales, et leurs mélanges.

3. Composition selon l'une quelconque des revendications précédentes, **caractérisée en ce que** les fibres sont des fibres d'origine synthétique.

4. Composition selon l'une quelconque des revendications précédentes, **caractérisée en ce que** les fibres sont des fibres de polyamide ou de poly-p-phénylène téréphtamide.

5. Composition selon l'une quelconque des revendications précédentes, **caractérisée en ce que** les fibres ont une longueur allant de 0,1 à 1,5 mm.

6. Composition selon l'une quelconque des revendications précédentes, **caractérisée en ce que** les fibres ont une section comprise dans un cercle de diamètre moyen allant de 5 à 50 µm.

7. Composition selon l'une quelconque des revendications précédentes, **caractérisée en ce que** les fibres sont présentes en une quantité allant de 0,1 à 20 % en poids par rapport au poids total de la composition.

8. Composition selon l'une quelconque des revendications précédentes, **caractérisée en ce que** le tensioactif siliconé est choisi parmi les alkyldiméthicone copolyols.

9. Composition selon l'une quelconque des revendications précédentes, **caractérisée en ce que** la quantité de tensioactif siliconé va de 0,1 à 5 % en poids de matière active par rapport au poids total de la composition.

10. Composition selon l'une quelconque des revendications précédentes, **caractérisée par le fait que** la cire est choisie parmi les cires d'origine animale, les cires d'origine végétale, les cires minérales, les cires synthétiques, les cires de silicone, les huiles hydrogénées concrètes à 25°C et les esters gras concrets à 25°C et leurs mélanges.

11. Composition selon l'une quelconque des revendications précédentes, **caractérisée par le fait que** la quantité de cire(s) va de 0,5 à 10 % en poids par rapport au poids total de la composition.

12. Composition selon l'une quelconque des revendications précédentes, **caractérisée par le fait que** la phase huileuse est présente en une quantité allant de 10 à 50 % en poids par rapport au poids total de la composition.

13. Composition selon l'une quelconque des revendications précédentes, **caractérisée par le fait que** la phase huileuse contient au moins une huile de silicone.

14. Composition selon l'une quelconque des revendications précédentes, **caractérisée par le fait qu'**elle constitue une composition cosmétique ou dermatologique.

15. Utilisation cosmétique de la composition selon l'une quelconque des revendications 1 à 14, pour le traitement, la protection, le soin, le démaquillage et/ou le nettoyage de la peau, des lèvres et/ou des cheveux, et/ou pour le maquillage de la peau et/ou des lèvres.

16. Procédé de traitement cosmétique de la peau, y compris du cuir chevelu, des cheveux, et/ou des lèvres, **caractérisé par le fait que** l'on applique sur la peau, les cheveux et/ou les lèvres, une composition selon l'une quelconque des revendications 1 à 14.

## Patentansprüche

1. Zusammensetzung, die in Form einer Wasser-in-Öl-Emulsion (W/O) vorliegt und die in einem physiologisch akzeptablen Medium eine in einer Ölphase dispergierte wäßrige Phase enthält, **dadurch gekennzeichnet, daß** sie Fasern, mindestens ein grenzflächenaktives Silicon und mindestens ein Wachs enthält.

2. Zusammensetzung nach dem vorhergehenden Anspruch, **dadurch gekennzeichnet, daß** die Fasern unter den Fasern aus Seide, Baumwolle, Wolle, Leinen, Cellulosefasern, die insbesondere aus Holz, Gemüse oder Algen gewonnen wurden, Fasern aus Polyamid, Reyon, Viscose, Acetat und insbesondere dem Acetat von Reyon, Cellulose oder Seide, Poly-p-phenylenterephthamid, Acrylfasern, insbesondere aus Polymethylmethacrylat oder Poly-2-hydroxyethylmethacrylat, Fasern aus Polyolefin und insbesondere Polyethylen oder Polypropylen, Glas, Siliciumdioxid, Aramid, Kohlenstoff und insbesondere Kohlenstoff in Form von Graphit, Teflon®, unlöslichem Kollagen, Polyestern, Polyvinylchlorid, Polyvinylidenchlorid, Polyvinylalkohol, Polyacrylnitril, Chitosan, Polyurethan und Polyethylenphthalat, Fasern aus Polymergemischen, chirurgischen Fasern und deren Gemischen ausgewählt sind.

3. Zusammensetzung nach einem der vorhergehenden Ansprüche, **dadurch gekennzeichnet, daß** es sich bei den Fasern um Fasern synthetischer Herkunft handelt.

4. Zusammensetzung nach einem der vorhergehenden Ansprüche, **dadurch gekennzeichnet, daß** die Fasern Polyamidfasern oder Fasern aus Poly-p-phenylenterephthamid sind.

5. Zusammensetzung nach einem der vorhergehenden Ansprüche, **dadurch gekennzeichnet, daß** die Fasern eine Länge von 0,1 bis 1,5 mm aufweisen.

6. Zusammensetzung nach einem der vorhergehenden Ansprüche, **dadurch gekennzeichnet, daß** die Fasern einen Durchmesser aufweisen, der in einem Kreis mit einem mittleren Durchmesser von 5 bis 50 µm einbeschrieben werden kann.

7. Zusammensetzung nach einem der vorhergehenden Ansprüche, **dadurch gekennzeichnet, daß** die Fasern in einem Mengenanteil von 0,1 bis 20 Gew.-%, bezogen auf das Gesamtgewicht der Zusammensetzung, vorliegen.

8. Zusammensetzung nach einem der vorhergehenden Ansprüche, **dadurch gekennzeichnet, daß** das grenzflächenaktive Silicon unter den Alkyldimethiconcopolyolen ausgewählt ist.

9. Zusammensetzung nach einem der vorhergehenden Ansprüche, **dadurch gekennzeichnet, daß** das grenzflächenaktive Silicon in einem Mengenanteil von 0,1 bis 5 Gew.-% Wirkstoff, bezogen auf das Gesamtgewicht der Zusammensetzung, vorliegt.

10. Zusammensetzung nach einem der vorhergehenden Ansprüche, **dadurch gekennzeichnet**, das Wachs unter den Wachsen tierischer Herkunft, den Wachsen pflanzlicher Herkunft, den Mineralwachsen, den synthetischen Wachsen, den Siliconwachsen, den bei 25 °C festen hydrierten Ölen und den bei 25 °C festen Fettsäureestern und deren Gemischen ausgewählt ist.

11. Zusammensetzung nach einem der vorhergehenden Ansprüche, **dadurch gekennzeichnet, daß** der Mengenanteil des Wachses (der Wachse) im Bereich von 0,5 bis 10 Gew.-%, bezogen auf das Gesamtgewicht der Zusammensetzung, liegt.

12. Zusammensetzung nach einem der vorhergehenden Ansprüche, **dadurch gekennzeichnet, daß** die Ölphase in einer Menge von 10 bis 50 Gew.-%, bezogen auf das Gesamtgewicht der Zusammensetzung, vorliegt.

13. Zusammensetzung nach einem der vorhergehenden Ansprüche, **dadurch gekennzeichnet, daß** die Ölphase mindestens ein Siliconöl enthält.

14. Zusammensetzung nach einem der vorhergehenden Ansprüche, **dadurch gekennzeichnet, daß** es sich um eine kosmetische oder dermatologische Zusammensetzung handelt.

15. Kosmetische Verwendung der Zusammensetzung nach einem der Ansprüche 1 bis 14 zur Behandlung, zum Schutz, zur Pflege, zum Abschminken und/oder zur Reinigung der Haut, der Lippen und/oder der Haare und/oder zum Schminken der Haut und/oder der Lippen.

16. Verfahren zur kosmetischen Behandlung der Haut einschließlich der Kopfhaut, der Haare und/oder der Lippen, **dadurch gekennzeichnet, daß** auf die Haut, die Haare und/oder die Lippen eine Zusammensetzung nach einem der Ansprüche 1 bis 14 aufgetragen wird.

## Claims

1. Composition in the form of a water-in-oil (W/O) emulsion comprising, in a physiologically acceptable medium, an aqueous phase dispersed in an oily phase, **characterized in that** it contains fibres, at least one silicone surfactant and at least one wax.

2. Composition according to the preceding claim, **characterized in that** the fibres are chosen from silk, cotton, wool or flax fibres, cellulose fibres extracted in particular from wood, plants or algae, polyamide, rayon or viscose fibres, acetate fibres, in particular rayon acetate, cellulose acetate or silk acetate fibres, poly-p-phenylene terephthamide fibres, acrylic fibres, in particular polymethyl methacrylate or poly-2-hydroxyethyl methacrylate fibres, polyolefin fibres and in particular polyethylene or polypropylene fibres, glass, silica or aramid fibres, carbon fibres, in particular in graphite form, Teflon®, insoluble collagen, polyester, polyvinyl chloride or polyvinylidene chloride, polyvinyl alcohol, polyacrylonitrile, chitosan, polyurethane or polyethylene phthalate fibres, fibres from mixtures of polymers, surgical fibres, and mixtures thereof.

3. Composition according to either of the preceding claims, **characterized in that** the fibres are fibres of synthetic origin.

4. Composition according to any one of the preceding claims, **characterized in that** the fibres are polyamide fibres or poly-p-phenylene terephthamide fibres.

5. Composition according to any one of the preceding claims, **characterized in that** the fibres have a length ranging from 0.1 to 1.5 mm.

6. Composition according to any one of the preceding claims, **characterized in that** the fibres have a section comprised in a circle having an average diameter ranging from 5 to 50 µm.

7. Composition according to any one of the preceding claims, **characterized in that** the fibres are present in an amount ranging from 0.1 to 20% by weight relative to the total weight of the composition.

8. Composition according to any one of the preceding claims, **characterized in that** the silicone surfactant is chosen from alkyldimethicone copolyols.

9. Composition according to any one of the preceding claims, **characterized in that** the amount of silicone surfactant ranges from 0.1 to 5% by weight of active material relative to the total weight of the composition.

10. Composition according to any one of the preceding claims, **characterized in that** the wax is chosen from waxes of animal origin, waxes of plant origin, mineral waxes, synthetic waxes, silicone waxes, hydrogenated oils that are solid at 25°C and fatty esters that are solid at 25°C, and mixtures thereof.

11. Composition according to any one of the preceding claims, **characterized in that** the amount of wax(es) ranges from 0.5 to 10% by weight relative to the total weight of the composition.

12. Composition according to any one of the preceding claims, **characterized in that** the oily phase is present in an amount ranging from 10 to 50% by weight relative to the total weight of the composition.

13. Composition according to any one of the preceding claims, **characterized in that** the oily phase contains at least one silicone oil.

14. Composition according to any one of the preceding claims, **characterized in that** it constitutes a cosmetic or dermatological composition.

15. Cosmetic use of the composition according to any one of Claims 1 to 14, for treating, protecting, caring for, removing make-up from and/or cleansing the skin, the lips and/or the hair, and/or for making up the skin and/or the lips.

16. Cosmetic treatment process for the skin, including the scalp, the hair and/or the lips, **characterized in that** a composition according to any one of Claims 1 to 14 is applied to the skin, the hair and/or the lips.
